Europäisches Patentamt

European Patent Office

Office européen des brevets

(19)

(11) Numéro de publication: **0 240 376**
**B1**

(12) **FASCICULE DE BREVET EUROPEEN**

(45) Date de publication du fascicule du brevet:
**20.06.90**

(51) Int. Cl.⁵: **A61B 17/56**

(21) Numéro de dépôt: **87400332.0**

(22) Date de dépôt: **16.02.87**

(54) Dispositif d'immobilisation d'un élément osseux, notamment pour intervention orthopédique.

(30) Priorité: **28.02.86 FR 8602818**

(43) Date de publication de la demande:
**07.10.87 Bulletin 87/41**

(45) Mention de la délivrance du brevet:
**20.06.90 Bulletin 90/25**

(84) Etats contractants désignés:
**BE DE GB IT SE**

(56) Documents cités:
**EP-A- 0 146 872**
**FR-A- 2 107 676**
**GB-A- 136 297**
**GB-A- 1 562 061**
**GB-A- 2 099 904**
**GB-A- 2 107 990**
**GB-A- 2 114 891**

(73) Titulaire: **Hardy, Jean-Marie, Chateau de Noailles,
F-19600 Larches(FR)**

(72) Inventeur: **Hardy, Jean-Marie, Chateau de Noailles,
F-19600 Larches(FR)**

(74) Mandataire: **Hirsch, Marc-Roger, Cabinet Hirsch 34 rue
de Bassano, F-75008 Paris(FR)**

ACTORUM AG

## Description

La présente invention est relative à un dispositif d'immobilisation et de fixation externe d'un élément osseux en particulier d'un membre humain, notamment pour la mise en oeuvre de la méthode d'intervention dite d'Ilizarov.

On sait que cette méthode, classique dans le domaine de la chirurgie orthopédique, consiste à associer, à un élément osseux ou à deux parties d'un tel élément, des aiguilles ou broches croisées, traversant deux à deux l'élément ou la structure osseuse et supportées dans des positions exactement définies par une sorte de boîtier externe ajouré, comprenant deux arceaux ou parties d'arceaux métalliques, rigides, entourant l'élément osseux et séparés l'un de l'autre par des organes d'entretoisement à longueur réglable les maintenant en position fixe à distance appropriée, les aiguilles ou broches traversant la structure osseuse étant assujetties à ces arceaux ou parties d'arceaux et immobilisées sur ceux-ci pour coopérer avec l'élément osseux en deux zones distinctes, séparées et parfaitement localisées. Avantageusement, les broches ou aiguilles sont engagées dans des supports portés par les arceaux et disposées de telle sorte qu'elles soient orientées selon des rayons des arceaux, ces supports comprenant en outre des moyens propres à exercer une tension ou une compression sur ces broches ou aiguilles pour appliquer à l'élément osseux un effort approprié.

Dans certaines réalisations de tels dispositifs d'immobilisation utilisés pour la mise en oeuvre de la méthode d'Ilizarov, notamment du genre de celles décrites dans FR-A 2 577 793 publié le 29/08.86 pour un dispositif de fixateur externe à usage orthopédique, on a prévu, en particulier, de constituer les arceaux au moyen d'un tube à section circulaire sur lequel sont engagés des colliers articulés, munis de moyens d'immobilisation en toute position choisie sur ces arceaux, certains de ces colliers comportant en outre des moyens de fixation des entretoises, ainsi que des moyens propres à recevoir et immobiliser les extrémités des broches ou aiguilles dont les extrémités opposées coopèrent avec des organes de mise sous tension réglable de ces broches.

Or, cette solution, qui offre divers avantages, notamment quant à sa simplicité de réalisation et au fait qu'elle comporte un nombre réduit de pièces nécessaires à sa mise en oeuvre, permettant ainsi de réaliser un positionnement relativement précis des aiguilles ou broches vis-à-vis de l'élément osseux à traverser, présente néanmoins des inconvénients. En particulier, dans cette solution, une fois les arceaux immobilisés en position après réglage de la longueur de leurs entretoises d'écartement, il n'est pas possible de modifier ni l'orientation en azimuth des broches ou aiguilles traversantes, celles-ci restant toujours orientées selon les rayons de ces arceaux, ni leur positionnement en hauteur, les colliers de fixation étant directement traversés par ces broches ou aiguilles dont ils sont solidarisés.

De plus, du fait de la section arrondie des arceaux, le serrage d'un collier sur ceux-ci pour son immobilisation, ne permet pas toujours, sous l'effet du couple de rotation créé lorsqu'un effort de tension ou de compression est exercé sur la broche ou aiguille portée par ce collier, d'éviter que celui-ci ne tourne sur l'arceau en décalant alors, même légèrement, l'orientation de la broche ou de l'aiguille correspondante. Il en résulte un défaut dont les conséquences peuvent être particulièrement préjudiciables lors d'une réduction d'une fracture ou lors d'un traitement de cette dernière par ostéosynthèse.

La présente invention vise des perfectionnements apportés à un dispositif d'immobilisation d'un élément osseux du genre rappelé ci-dessus, en particulier du type de celui décrit dans la demande de brevet précitée, qui, en palliant les inconvénients de la solution décrite, apporte des avantages complémentaires à ceux qui résultent déjà de la mise en oeuvre de la structure ainsi envisagée.

Selon l'invention, le dispositif considéré se caractérise en ce que chaque arceau présente en section droite un contour externe présentant deux méplats parallèles, raccordés respectivement par deux portions convexes, les supports des broches ou aiguilles portées par l'arceau étant constitués par des cavaliers ouverts latéralement et présentant deux ailes comportant des surfaces planes aptes à coopérer par glissement plan sur plan avec les méplats de l'arceau, ces ailes étant réunies par une portion concave épousant le profil d'une des portions convexes du contour externe de l'arceau.

Grâce à ces dispositions, les cavaliers de support des broches ou aiguilles s'emboîtent aisément par le côté latéral de l'arceau destiné à les supporter et, une fois mis en place, ne peuvent plus subir de rotation relative autour de l'arceau correspondant lors des efforts exercés sur ceux-ci, résultant de la mise sous tension ou en compression de ces broches ou aiguilles, la portée plan sur plan des ailes des cavaliers sur les méplats de l'arceau empêchant tout décalage de l'un par rapport à l'autre dans le plan contenant la broche ou l'aiguille supportée par le cavalier.

Selon une caractéristique particulière, chaque cavalier monté sur un arceau comporte dans une de ses ailes coopérant avec un des méplats de l'arceau, un alésage transversal, s'étendant parallèlement au méplat, taraudé intérieurement et dans lequel est apte à être manoeuvrée une vis dont l'extrémité fait saillie dans un trou perpendiculaire à l'alésage, prévu à l'extrémité de l'aile du cavalier et à l'intérieur duquel est montée une bille d'arrêt faisant saillie à l'extrémité de ce trou pour déboucher à travers un orifice de plus faible diamètre délimité par un rétrécissement du trou, de telle sorte que le déplacement de la vis assure le blocage de la bille contre l'arceau et l'immobilisation du cavalier.

Selon une autre caractéristique de l'invention, au moins une aile de chaque cavalier présente extérieurement un tenon ou une mortaise, apte à s'engager respectivement dans une mortaise ou un tenon de même profil, ménagé dans un support comprenant un passage traversé par un fourreau fileté extérieurement, lui-même muni axialement d'un canal étroit pour la pénétration d'une broche ou aiguille immobilisée par rapport au fourreau, le support comportant par ailleurs un logement transversal dans lequel est monté prisonnier un écrou coopérant avec le filetage externe du fourreau tandis que le passage de celui-ci comporte, intérieurement, une clavette coulissant dans une rainure axiale prévue dans la surface externe du fourreau, de telle sorte que la rotation de l'écrou provoque le déplacement du fourreau dans le support et la mise sous tension ou sous compression par rapport à l'élément osseux de la broche ou de l'aiguille correspondante. Selon une caractéristique complémentaire, la broche ou aiguille est immobilisée dans le canal étroit du fourreau au moyen d'une vis engagée dans un alésage radial d'une tête rapportée sur l'extrémité du fourreau.

Selon une autre caractéristique avantageuse de l'invention et en fonction notamment des nécessités particulièrement utiles d'adjustement en hauteur de chaque support sur un des arceaux du dispositif, chaque cavalier est associé à une cale rapportée sur une des ailes du cavalier, la liaison entre la cale et le cavalier étant réalisée au moyen d'un axe porté par la cale et engagé dans un alésage prévu dans l'aile du cavalier, cet alésage s'étendant perpendiculairement aux méplats de l'arceau. De préférence, l'immobilisation de la cale par rapport au cavalier est réalisée par une vis de blocage radiale, traversant un trou fileté prévu dans le corps du cavalier et débouchant dans l'alésage de passage de l'axe porté par la cale. Utilement, l'axe présente une gorge intermédiaire dans laquelle pénètre l'extrémité de la vis de blocage.

Selon encore une autre caractéristique du dispositif de l'invention, chaque arceau est avantageusement constitué au moyen de deux demi-arceaux, aptes à être réunis conjointement par un jeu de deux prolongateurs rectilignes, de longueur variable dans chaque jeu, présentant en section droite un profil identique à celui de l'arceau, chaque prolongateur comportant à ses extrémités des organes de liaison avec les extrémités en regard de chaque demi-arceau. De préférence, chaque organe de liaison est constitué par un embout saillant, porté par le demi-arceau ou le prolongateur, et pénétrant dans un logement de même profil ménagé dans le prolongateur ou le demi-arceau respectivement, le blocage du prolongateur et du demi-arceau après engagement de l'embout dans son logement étant réalisé par au moins une vis transversale.

Selon une autre caractéristique également, chacun des arceaux est aussi associé à un ensemble de colliers doubles, constitués chacun de deux paires de mâchoires, réunies par un axe transversal commun, l'une de ces paires de mâchoires étant apte à serrer l'arceau et l'autre l'extrémité d'une entretoise d'écartement ajustable, montée entre les deux arceaux parallèles et s'étendant perpendiculairement au plan de ces arceaux. Avantageusement, chaque entretoise est constituée de deux parties disposés dans le prolongement l'une de l'autre et associées à un ridoir intermédiaire, comprenant une tige dont les deux extrémités opposées sont filetées en sens inverse et coopèrent respectivement avec deux écrous solidaires des extrémités en regard des deux parties de l'entretoise.

Selon une autre caractéristique particulière, une des deux paires de mâchoires du collier double peut être rendue solidaire d'une barre comportant des passages successifs pour la fixation d'un support de broche ou d'aiguille, disposé en porte à faux vis-à-vis de l'arceau.

D'autres caractéristiques d'un dispositif d'immobilisation établi conformément à l'invention apparaîtront encore à travers la description qui suit de plusieurs exemples de réalisation, donnés à titre indicatif et non limitatif, en référence aux dessins annexés sur lesquels:

- la figure 1 est une vue en perspective d'un dispositif de fixation selon l'invention, représenté immobilisant un élément osseux allongé, monté dans celui-ci;
- la figure 2 est une vue en coupe transversale à plus grande échelle du support d'une aiguille ou d'une broche de traversée de l'élément osseux porté par le dispositif;
- la figure 3 est une vue en perspective d'un cavalier entrant dans la réalisation du support de broche ou aiguille, selon la figure 2;
- la figure 4 est une vue en coupe transversale du cavalier illustré sur la figure 3;
- la figure 5 est une vue de dessus à plus petite échelle d'un arceau de fixation du dispositif selon la figure 1, représenté constitué de deux demi-arceaux réunis par des éléments prolongateurs selon diverses variantes de réalisation;
- la figure 6 est une vue en coupe d'un des éléments prolongateurs, illustré sur la figure 5;
- la figure 7 est une vue en élévation et en coupe partielle d'une entretoise utilisée dans le dispositif selon la figure 1 pour maintenir un écartement déterminé entre les deux arceaux de fixation;
- la figure 8 est une vue de détail d'un collier double entrant dans la réalisation du dispositif permettant le montage d'une barre pour le support en porte à faux d'aiguilles ou broches de fixation.

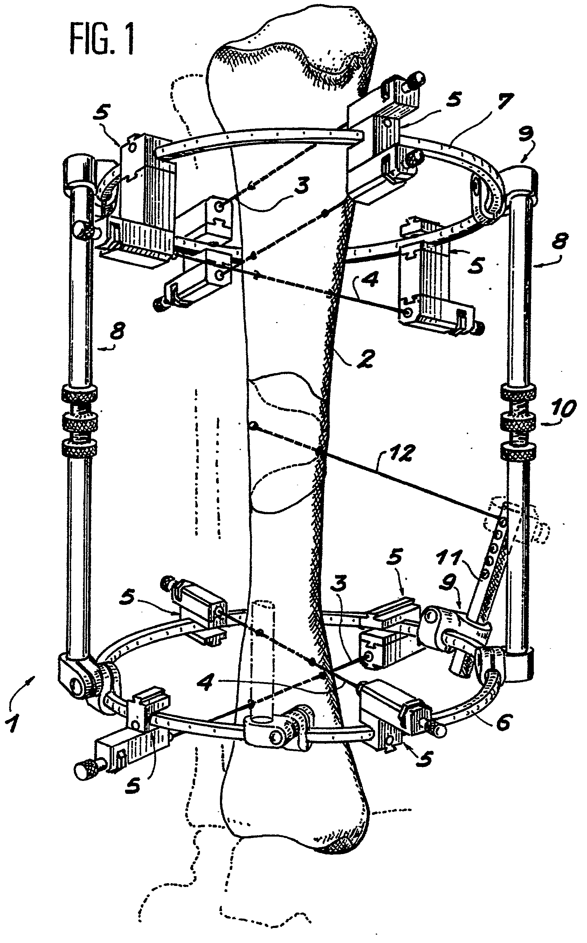

Sur la vue en perspective illustrée sur la figure 1, le dispositif de fixation selon l'invention est désigné dans son ensemble sous la référence 1 et est plus particulièrement destiné à l'immobilisation d'un élément osseux 2, représenté en position verticale à l'intérieur du dispositif.

Pour immobiliser l'élément 2, le dispositif comporte conformément à une disposition connue et mise en oeuvre de façon classique dans le méthode de traitement dite d'Ilizarov, deux jeux de deux aiguilles ou broches, traversant respectivement les extrémités de l'élément osseux 2 et désignés sur le dessin dans chaque jeu sous les références 3 et 4. Chaque aiguille 3 ou 4 est immobilisée à l'intérieur d'un support 5 dont le détail de réalisation sera décrit plus loin, les supports étant montés respectivement sur deux arceaux 6 et 7 s'étendant selon deux plans sensiblement parallèles et disposés au voisinage des extrémités de l'élément osseux 2 afin de permettre au dispositif selon l'invention d'assurer l'immobilisation convenable de ce dernier.

Les arceaux 6 ou 7 sont maintenus à une distance d'écartement convenable par l'intermédiaire d'entretoises 8, ajustables, les extrémités de ces entretoises étant réunies par des colliers doubles 9 aux arceaux 6 et 7 respectivement. Notamment, chaque entretoise est de préférence formée de deux parties disposées dans le prolongement l'une de l'autre et comporte un organe 10, formant ridoir permettant de régler l'écartement de ces deux parties et la longueur de l'entretoise correspondante pour permettre de faire varier à la demande la distance qui sépare les deux arceaux 6 et 7. D'autres colliers 9 sont également montés sur les arceaux 6 et 7 afin d'assurer l'immobilisation de barres de support telles que 11, disposées en porte à faux vis-à-vis de ces colliers et permettant le maintien d'autres broches ou aiguilles 12, traversant cette fois obliquement l'élément osseux 2, ces aiguilles complémentaires 12 étant généralement disposées entre les plans des arceaux parallèles 6 et 7.

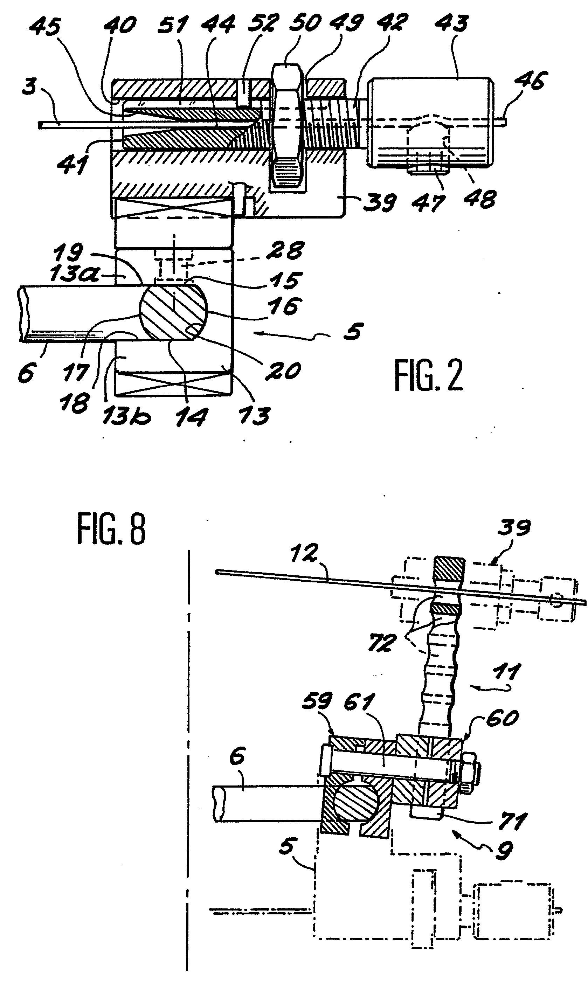

La figure 2 illustre à plus grande échelle le détail partiel de la réalisation d'un élément de support 5, monté par exemple sur l'un des arceaux 6 du dispositif. Chaque support 5 comporte ainsi un cavalier 13, apte à s'emboîter par ses deux ailes 13a et 13b sur deux méplats 14 et 15 prévus sur l'arceau 6, ces méplats 14 et 15 étant réunis l'un à l'autre par des portions convexes 16 et 17 sensiblement demi-cylindriques. A cet effet, le cavalier 13 présente un logement ouvert délimité par les deux ailes 13a et 13b, dont les surfaces planes 18 et 19 sont aptes à coopérer par glissement plan sur plan avec les méplats 14 et 15, les surfaces 18 et 19 étant elles-mêmes réunies par une portion concave 20 dont le profil est apte à épouser sensiblement celui de la portion convexe 16 réunissant, dans l'arceau 6, les méplats 14 et 15.

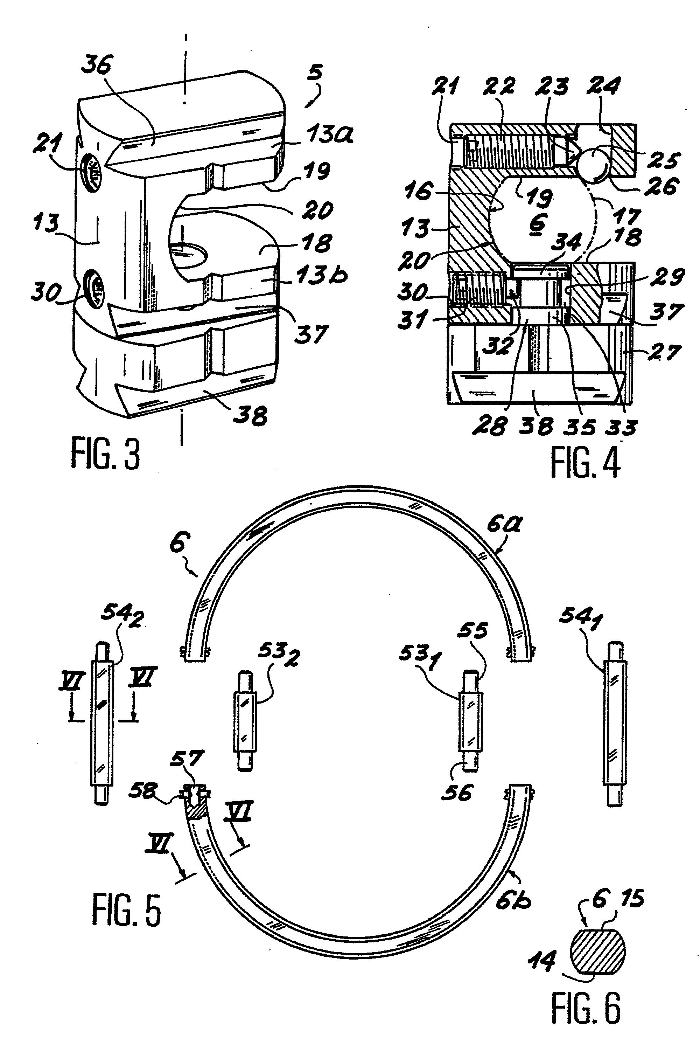

Comme on le voit plus particulièrement sur les figures 3 et 4, chaque cavalier 13 comporte avantageusement dans l'une de ses ailes un alésage transversal 21 à l'intérieur duquel est montée une vis 22, apte à coopérer avec un filetage convenablement usiné à l'intérieur de l'alésage, cette vis 22 présentant une extrémité 23 débouchant à l'intérieur d'un trou 24 s'étendant perpendiculairement à l'alésage 21. A l'intérieur de ce trou 24 est montée une bille prisonnière 25 qui déborde légèrement d'un orifice 26 délimité par un rétrécissement correspondant du trou 24 et prévu à l'extrémité inférieure de celui-ci en direction de l'aile 13a du cavalier 13 sous laquelle s'engage le méplat correspondant de l'arceau 6, cette bille 25 poussée vers le bas par l'extrémité 23 de la vis 22 permettant, une fois le cavalier convenablement engagé sur l'arceau et notamment avec ses surfaces planes 18 et 19 en appui glissant sur les méplats 14 et 15, de bloquer ce cavalier comme illustré plus particulièrement sur la figure 4.

Selon l'invention, chacun des cavaliers 13 ou seulement certains de ceux-ci peuvent être avantageusement associés à une cale d'épaisseur 27, apte à être fixée sur l'une des ailes du cavalier correspondant au moyen d'un pion ou d'un axe transversal 28 s'engageant dans un passage 29 prévu dans l'une des ailes du cavalier. Celui-ci comporte par ailleurs un second alésage transversal 30, parallèle à l'alésage 21 et dans lequel est montée une vis 31 présentant en bout un téton 32 d'immobilisation de l'axe 28 par rapport au cavalier, en s'engageant dans une gorge intermédiaire 33 prévue entre deux rebords parallèles 34 et 35 usinés sur l'axe 28. Chacun des cavaliers 13 comporte dans l'une et/ou l'autre de ses ailes 13a et 13b, des mortaises 36 et 37 aptes à permettre la fixation d'un bloc 39 pour l'immobilisation des broches ou aiguilles traversantes 3 ou 4, destinées à immobiliser l'élément osseux 2. De même, chaque cale 27 comporte également, dans sa face opposée à celle appliquée contre le cavalier 13, une mortaise analogue 38, pour le cas où le bloc 39 est à monter sur le cavalier après mise en place de cette cale, pour en ajuster convenablement la hauteur.

En se référant à nouveau à la figure 2, on voit que le cavalier 13, muni le cas échéant de la cale 27, peut être ainsi associé à un tel bloc support 39, qui comporte un alésage 40 à l'intérieur duquel est monté un fourreau 41. Celui-ci présente, sur sa surface extérieure, un filetage 42 et se termine par une tête de blocage 43. Ce fourreau 41 comporte également un canal axial 44 présentant, à son extrémité opposée à la tête 43, une ouverture évasée 45, permettant l'engagement de l'une des aiguilles traversantes, par exemple l'aiguille 3, cette dernière qui se prolonge en direction de la tête 43 étant immobilisée à l'intérieur de celle-ci par son extrémité 46 au moyen d'une vis de blocage 47 engagée dans un trou fileté transversal 48 prévu dans la tête 43.

Le bloc support 39 comporte par ailleurs un logement médian 49, à l'intérieur duquel est monté un écrou prisonnier 50 coopérant avec le filetage 42 du fourreau 41. Ce fourreau 41 comporte en outre une rainure longitudinale 51 dans laquelle est apte à coulisser une clavette 52, de telle sorte que le mouvement de rotation de l'écrou 50 sur le filetage 42 provoque le déplacement axial du fourreau 41 à l'intérieur de l'alésage 40, en permettant ainsi d'exercer, sur la broche ou l'aiguille 3 traversant l'élément osseux 2, un effort de traction ou de compression approprié.

Les figures 5 et 6 illustrent un autre détail de réalisation du dispositif d'immobilisation selon l'invention, concernant la structure particulière de chacun des arceaux 6 ou 7 permettant d'assurer la fixation des supports 5 des aiguilles traversantes 3 et 4.

Dans la vue représentée sur ces figure, l'arceau inférieur 6 par exemple est constitué de deux demi-arceaux 6a et 6b, réunis l'un à l'autre par un jeu de prolongateurs, tels que $53_1$ et $53_2$ ou $54_1$ et $54_2$, chaque paire de prolongateurs présentant la même dimension longitudinale. Ces prolongateurs comportent chacun des embouts d'extrémité, respectivement 55 et 56, aptes à s'engager dans des logements tels que 57 prévus à l'intérieur des demi-arceaux 6a et 6b dans leurs extrémités en regard, ces prolongateurs étant en outre susceptibles d'être immobilisés, une fois montés sur les demi-arceaux, au moyen de vis de blocage latérales 58, coopérant avec les logements 57 précédents.

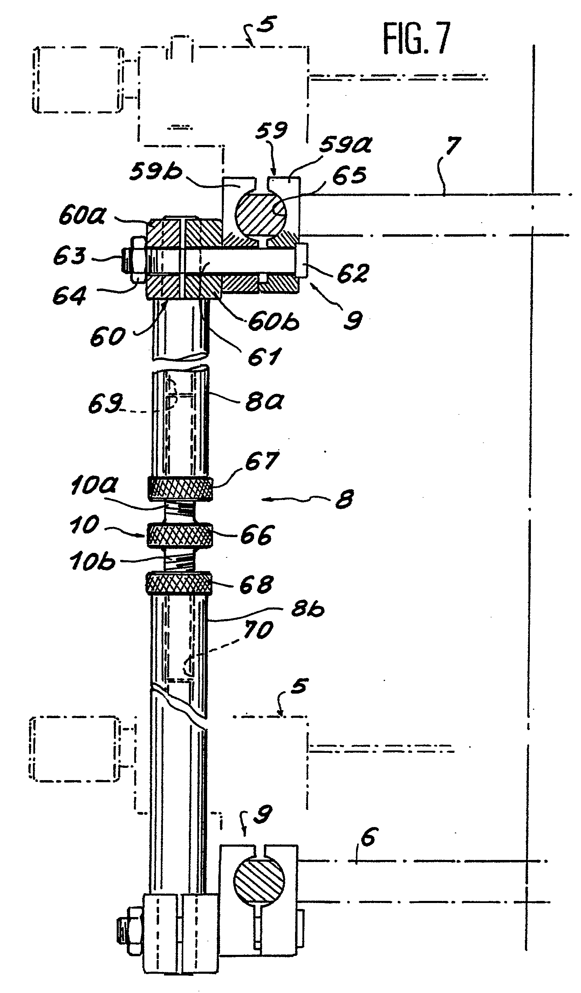

La figure 7 illustre par ailleurs plus particulièrement le détail de la réalisation des colliers doubles 9, assurant le montage sur les arceaux 6 et 7 des entretoises 8 et des organes 10 qui permettent d'assurer l'ajustement en longueur de ces entretoises et par suite l'écartement approprié de ces arceaux par rapport à la longueur de l'élément 2.

Notamment, chaque collier double 9 est ici constitué de deux éléments voisins, respectivement 59 et 60, formés de deux paires de mâchoires 59a et 59b d'une part, 60a et 60b d'autre part, ces éléments étant réunis entre eux par un axe commun 61, comportant une tête d'arrêt 62 et une extrémité filetée 63, opposée à la tête 62 et sur laquelle est engagé un écrou de blocage 64. Chaque paire de mâchoires, par exemple la paire 59a et 59b, délimite un logement de forme sensiblement circulaire 65, apte à venir par exemple s'engager sur les portions convexes 16 et 17 de l'un des arceaux 6 ou 7, l'autre paire de mâchoires 60a et 60b coopérant alors directement avec l'extrémité correspondante de l'entretoise 8. En outre, et afin de permettre l'ajustement en longueur du montage et de régler la distance qui sépare les arceaux 6 et 7, cette entretoise est formée de deux parties 8a et 8b, disposées dans le prolongement l'une de l'autre et à l'extrémité desquelles sont soudés deux écrous 67 et 68 coopérant avec les extrémités filetés 10a et 10b de l'organe de réglage 10 correspondant. Celui-ci comporte notamment un bouton moleté 66, permettant de faire tourner l'organe 10 à l'intérieur de deux logements 69 et 70 prévus dans les parties 8a et 8b de l'entretoise, les extrémités 10a et 10b présentant des filetages de sens inverse qui, en tournant dans les écrous 67 et 68, réalisent à volonté l'écartement ou le rapprochement des deux parties 8a et 8b et, par suite, ajustent la position relative des arceaux 6 et 7.

La figure 8 illustre enfin un autre détail de la réalisation du dispositif d'immobilisation selon l'invention dans lequel certains des colliers doubles 9 montés sur l'un des arceaux 6 ou 7 comportant, comme précédemment, deux éléments de fixation 59 et 60, sont aménagés de telle sorte que le premier de ceux-ci soit immobilisé sur l'arceau correspondant tandis que le second assure le blocage en position d'une barre 11 disposée en porte à faux et dont une extrémité 71 est bloquée dans la paire de mâchoires de l'élément 60, cette barre comportant elle-même une série de trous transversaux 72 pour le montage en position réglable de supports 39 du genre de ceux déjà décrits. Ces supports 39 immobilisent à nouveau une broche ou aiguille 12, apte à traverser l'élément osseux 2 dans une partie sensiblement médiane de ce dernier, de la façon illustrée sur la figure 1, avec en particulier une orientation inclinée sur la verticale ou plus généralement sur la direction longitudinale de l'élément 2, pour exercer sur ce dernier un effort de traction ou de compression complémentaire nécessaire au traitement envisagé.

On réalise ainsi un dispositif d'immobilisation qui, tout en conservant les avantages de celui déjà décrit et revendiqué dans la demande de brevet n° 85 02 577, en particulier en ce qui concerne la simplicité de mise en oeuvre et les facilités de pose et de dépose, apporte des perfectionnements substantiels en améliorant notamment les conditions de fixation et de blocage des aiguilles ou broches sur les arceaux, ainsi que les facultés du dispositif pour adapter aux supports de ces aiguilles des organes de réglage en position et en orientation. Les cavaliers, une fois montés sur les arceaux, ne peuvent plus tourner dans leur plan quel que soit l'effort exercé sur l'aiguille correspondante, du fait du profil à méplats prévu pour ces pièces. En outre, le montage préconisé pour les cales d'épaisseur sur ces cavaliers autour d'un axe de liaison permet d'autoriser, pour les supports des broches ou aiguilles, une rotation relative dans un plan sensiblement parallèle à celui de l'arceau portant les supports correspondants, afin notamment de permettre une meilleure permutation de ces aiguilles dans l'élément osseux immobilisé dans le dispositif.

**Revendications**

1.- Dispositif d'immobilisation d'un élément osseux, notamment pour intervention orthopédique, comprenant des aiguilles ou broches, traversant l'élément, montées dans des supports eux-mêmes portés par deux arceaux métalliques rigides entourant l'élément osseux et séparés l'un de l'autre par des entretoises à longueur réglable, caractérisé en ce que chaque arceau (6,7) présente en section droite un contour externe présentant deux méplats (14,15) parallèles, raccordés respectivement par deux portions convexes (16,17), les supports des broches ou aiguilles portées par l'arceau étant constitués par des cavaliers (13) qui présentent un logement ouvert latéralement délimité par deux ailes (13a,13b) comportant des surfaces planes (18-19), aptes à coopérer par glissement plan sur plan avec les méplats (14,15) de l'arceau, ces ailes étant réunies par une portion concave (20) épousant le profil d'une des portions convexes du contour externe de l'arceau.

2.- Dispositif d'immobilisation selon la revendication 1, caractérisé en ce que chaque cavalier (13), monté sur un arceau (6,7) comporte dans une de ses ailes coopérant avec un des méplats de l'arceau, un alésage transversal (21), s'étendant parallèlement au méplat, taraudé intérieurement, et dans lequel est apte à être manoeuvrée une vis (22) dont l'extrémité fait saillie dans un trou perpendiculaire à l'alésage, prévu à l'extrémité de l'aile du cavalier et à l'intérieur duquel est montée une bille d'arrêt (25), faisant saillie à l'extrémité de ce trou pour déboucher à travers un orifice (26) de plus faible diamètre, délimité par un rétrécissement du trou, de telle sorte que le déplacement de la vis assure le blocage de la bille contre l'arceau et l'immobilisation du cavalier (13).

3.- Dispositif d'immobilisation selon l'une des revendications 1 ou 2, caractérisé en ce qu'au moins une aile (13a, 13b) de chaque cavalier (13) présente extérieurement un tenon ou une mortaise (36,37), apte à s'engager respectivement dans une mortaise ou tenon de même profil, ménagé dans un support (39) comprenant un passage (40) où s'engage un fourreau (41) fileté extérieurement, lui-même muni axialement d'un canal étroit (45) pour la pénétration d'une broche ou aiguille (3,4) immobilisée par rapport au fourreau, le support comportant par ailleurs un logement transversal (49) dans lequel est monté prisonnier un écrou (50) coopérant avec le filetage (42) du fourreau tandis que le passage de celui-ci comporte, intérieurement, une clavette (52) coulissant dans une rainure axiale (51) prévue dans la surface externe du fourreau, de telle sorte que la rotation de l'écrou provoque le déplacement du fourreau dans le support et la mise sous tension ou sous compression par rapport à l'élément osseux (2) de la broche ou aiguille correspondante.

4.- Dispositif d'immobilisation selon la revendication 3, caractérisé en ce que la broche ou aiguille (3,4) est immobilisée dans le canal étroit (45) du fourreau (41) au moyen d'une vis (47) engagée dans un alésage radial (48) d'une tête (43) montée en bout du fourreau.

5.- Dispositif d'immobilisation selon l'une quelconque des revendications 1 à 4, caractérisé en ce que chaque cavalier (13) est apte à être associé à une cale (27) agencée pour être rapportée sur une des ailes (13a,13b) de ce cavalier, la liaison entre la cale (27) et le cavalier (13) étant réalisée au moyen d'une axe (28) porté par la cale et engagé dans un alésage (29) prévu dans une aile du cavalier et s'étendant perpendiculairement à la surface des méplats (14,15) de l'arceau (6,7).

6.- Dispositif d'immobilisation selon la revendication 5, caractérisé en ce que l'immobilisation de la cale (27) par rapport au cavalier (13) est réalisée par une vis de blocage radiale (31), traversant un trou fileté (30) prévu dans le corps du cavalier et débouchant dans l'alésage (29) de passage de l'axe (28) porté par la cale.

7.- Dispositif d'immobilisation selon la revendication 6, caractérisé en ce que l'axe (28) présente une gorge intermédiaire dans laquelle pénètre l'extrémité (32) de la vis de blocage (31).

8.- Dispositif d'immobilisation selon l'une quelconque des revendications 1 à 7, chaque arceau (6,7) étant constitué au moyen de deux demi-arceaux (6a-6b,), aptes à être réunis conjointement par un jeu de deux prolongateurs rectilignes (53,54), de longueur variable, caractérisé en ce que chaque prolongateur (53,54) présente en section droite un profil identique à celui de l'arceau, et comporte à ses extrémités des organes de liaison (55,56) avec les extrémités en regard de chaque demi-arceau (6a,6b).

9.- Dispositif d'immobilisation selon la revendication 8, caractérisé en ce que chaque organe de liaison est constitué par un embout saillant (55,56) porté par le demi-arceau (6a,6b) ou le prolongateur (53,54), pénétrant dans un logement (57) de même profil ménagé dans le prolongateur ou le demi-arceau respectivement, le blocage du prolongateur et du demi-arceau après engagement de l'embout dans son logement étant réalisé par au moins une vis transversale (58).

10.- Dispositif d'immobilisation selon l'une des revendication 1 à 9, caractérisé en ce que chacun des arceaux (6,7) est associé à un ensemble de colliers doubles (9) constitués chacun de deux paires de mâchoires (59, 60) réunies par un axe transversal commun (61), l'une de ces paires de mâchoires étant apte à serrer l'arceau et l'autre, une extrémité d'une entretoise d'écartement ajustable (8), montée entre les deux arceaux parallèles (6,7) et s'étendant perpendiculairement au plan de ces arceaux.

11.- Dispositif d'immobilisation selon la revendication 10, caractérisé en ce que chaque entretoise (8) est constituée de deux parties (8a-8b) disposées dans le prolongement l'une de l'autre et associées à un ridoir intermédiaire (10), comprenant une tige dont les deux extrémités opposées (10a-10b) sont filetées en sens inverse et coopèrent respectivement avec deux écrous (67-68) solidaires des extrémités en regard des deux parties de l'entretoise (8).

12.- Dispositif d'immobilisation selon la revendication 11, caractérisé en ce qu'un de deux paires de mâchoires du collier double est rendue solidaire d'une barre (11) comportant des passages successifs (72) de fixation d'un support (39) de broche ou d'aiguille disposé en porte à faux vis-à-vis de l'arceau (6,7).

**Patentansprüche**

1. Vorrichtung zum Festlegen eines Knochenteils, insbesondere zwecks orthopädischer Operation, mit Nadeln oder Stiften, die den Knochenteil durchqueren und an Haltern angeordnet sind, welche von zwei steifen metallischen Reifen gehalten werden, die den Knochenteil umgeben und voneinander durch Querstreben einstellbarer Länge getrennt sind, dadurch gekennzeichnet, dass jeder Reifen (6, 7) im Querschnitt ein Profil mit zwei parallelen, durch zwei konvexe Teile (16, 17) miteinander verbundenen Flachteilen (14, 15) besitzt, wobei die von Reifen getragenen Stift- bzw. Nadelhalter aus Reitern (13) bestehen, die eine seitlich offene, durch zwei mit den Reifenflachteilen (14, 15) flachgleitend zusammenwirkende, ebene Oberflächen (18–19) aufweisende, Schenkel (13a, 13b) begrenzte Aufnahmeausnehmung aufweisen, während die Schenkel durch einen konkaven Teil (20) miteinander verbunden sind, der dem Profil eines der konvexen Reifenprofilteile entspricht.

2. Vorrichtung nach Anspruch 1, dadurch gekennzeichnet, dass jeder auf einem Reifen (6, 7) sitzende Reiter (13) eine in einem seiner mit einem dem Reifenprofilflachteil zusammenwirkenden Schenkel eine zu dem Reifenflachteil parallele mit einem Innengewinde versehene Gewindequerbohrung (21) aufweist, in der eine Schraube einstellbar eingeschraubt ist, deren Ende in eine senkrecht zur Schraubenbohrung am Ende des Reiterschenkels angeordnetes Loch (24) hineinragt, welches eine Verriegelungskugel (25) enthält, die auf einer Seite aus einer durch eine Verengung des Loches begrenzten Öffnung (26) kleineren Durchmessers herausragt, derart, dass eine Verstellung der Schraube die Kugel durch Anpressen an den Reifen blockiert und den Reiter (13) festlegt.

3. Vorrichtung nach Anspruch 1 oder 2, dadurch gekennzeichnet, dass wenigstens einer der Schenkel (13a, 13b) jedes Reiters (13) an seiner Aussenseite einen Vorsprung bzw. eine Ausnehmung (36, 37) aufweist, der (die) mit einer Ausnehmung bzw. einem Vorsprung gleichen Profils eines Halteteils (39) zusammenwirkt, der einen Durchlass (40) aufweist, in welches ein mit einem Aussengewinde (42) versehenes Rohrstück (41) eingesetzt ist, das einen engen Axialkanal (45) zur Einführung eines Stiftes bzw. einer Nadel (3, 4) aufweist, der (bzw. die) in Bezug auf das Rohrstück festgelegt ist, wobei der Halteteil (39) ferner eine Querausnehmung (49) besitzt, in der eine mit dem Gewinde (42) des Rohrstücks zusammenwirkende Schraubenmutter (50) eingeschlossen ist, während der Durchlass (40) des Rohrstücks einen in einer Axialnut (51) der Aussenfläche des Rohrstückes gleitend geführten inneren Keil (52) umfängt, derart, dass eine Verdrehung der Schraubenmutter eine Bewegung des Rohrstücks im Halteteil und durch den betreffenden Stift, bzw. die betreffende Nadel die Ausübung einer Zug- oder Druckkraft auf den Knochenteil (2) bewirkt.

4. Vorrichtung nach Anspruch 3, dadurch gekennzeichnet, dass der Stift bzw. die Nadel (3, 4) in dem engen Axialkanal (45) des Rohrstücks (41) durch eine in einer radialen Bohrung (48) eines am Ende des Rohrstücks angeordneten Kopfes (43) sitzende Schraube (47) festgelegt ist.

5. Vorrichtung nach einem der Ansprüche 1 bis 4, dadurch gekennzeichnet, das jeder Reiter (13) derart ausgebildet ist, dass er mit einem an einem der Schenkel (13a, 13b) des betreffenden Reiters einbringbaren Keil (27) zusammenwirkt, wobei die Verbindung zwischen dem Keil (27) und dem Reiter (13) durch einen vom Keil gehaltenen, in einer Bohrung des Reiterschenkels angeordneten Druckbolzen (28) erfolgt, dessen Achse senkrecht zur Oberfläche der Profilflachteile (14, 15) des Reifens (6, 7) verläuft.

6. Vorrichtung nach Anspruch 5, dadurch gekennzeichnet, dass der Keil (27) in Bezug auf den Reiter (13) durch eine radiale Blockierschraube (31) festgelegt ist, die eine im Reiter angeordnete Gewindebohrung (30) durchsetzt, welche in die den von dem Keil gehaltenen Druckbolzen (28) aufnehmende Durchlassbohrung (29) mündet.

7. Vorrichtung nach Anspruch 6, dadurch gekennzeichnet, dass der Druckbolzen (28) eine Zwischennut (33) aufweist, in welche das Ende (32) der Blockierschraube (31) eingreift.

8. Vorrichtung nach einem der Ansprüche 1 bis 7, bei der jeder Reifen (6, 7) aus zwei Halbreifen (6a-6b) besteht, die vermittels zweier geradliniger Verlängerungsteile (53, 54) veränderlicher Länge miteinander verbindbar sind, dadurch gekennzeichnet, dass jeder Verlängerungsteil (53, 54) im Querschnitt ein dem Reifenquerschnitt gleiches Profil besitzt, und an seinen Enden Organe (55, 56) zu seiner Verbindung mit den jeweiligen gegenüberliegenden Halbreifenenden (6a, 6b) aufweist.

9. Vorrichtung nach Anspruch 8, dadurch gekennzeichnet, dass jedes Verbindungsorgan ein herausragendes, vom Halbreifen (6a, 6b) oder vom Verlängerungsteil (53, 54) getragenes Endstück (55, 56) aufweist, das in eine Aufnahmeausnehmung (57) gleichen Profiles des Halbreifens bzw. des Verlängerungsteils passt, wobei die Festlegung des Verlängerungsteils und des Halbreifens nach der Einführung des Endstücks in die zugehörige Aufnahmeausnehmung durch wenigstens eine Querschraube (58) erfolgt.

10. Vorrichtung nach einem der Ansprüche 1 bis 9, dadurch gekennzeichnet, dass jeder Reifen (6, 7) mit Doppelschellen (9) verbunden ist, die aus je zwei durch eine gemeinsame Querachse (61) miteinander verbundenen Spannbacken (59, 60) bestehen, wobei je ein Spannbackenpaar den Reifen, und das andere ein Ende eines einstellbaren Zwischenstücks (8) einklemmt, das zwischen den beiden parallelen Reifen (6, 7) senkrecht zu deren Ebenen angeordnet ist.

11. Vorrichtung nach Anspruch 10, dadurch gekennzeichnet, dass jedes Zwischenstück (8) aus zwei miteinander fluchtenden Teilen (8a-8b) besteht, die durch ein Verbindungsstück (10) miteinander verbunden sind, welches eine an ihren beiden Enden (10a-10b) mit entgegengesetzten Gewinden versehene Stange aufweist, deren Enden (10a-10b) mit zwei mit den Enden der jeweils einander gegenüberliegenden Zwischenstücke (8) fest verbundenen Schraubenmuttern (67, 68) zusammenwirken.

12. Vorrichtung nach Anspruch 11 dadurch gekennzeichnet, dass eines der zwei Spannbackenpaare der Doppelflanschanordnung mit einer Stange (11) fest verbunden ist, die aufeinanderfolgende Ausnehmungen (72) zur Aufnahme eines bezüglich des Reifens (6, 7) fliegend angebrachten Stift- oder Nadelhalters (39) aufweist.

## Claims

1. Holding device for immobilizing a bone element, particulary with a view to orthopedic surgical intervention, comprising needles or rods which, while extending through said bone element, are mounted on supports that are held by two rigid metallic ring members surrounding said bone element and separated from each other by spacing elements of adjustable length, characterized in that each ring member (6, 7) presents, as seen in cross-section, an outer profil defining two parallel flat portions (14, 15) connected, respectively, by two convex portions (16, 17), said supports which hold said rods or needles and which are maintained by the ring member being constituted by riders (13) defining a laterally open recess delimited by two flanges (13a, 13b) with planar surfaces (18-19) adapted to cooperate slidingly and in a coplanar manner with said flat portions (14, 15) of the ring member, said flanges being connected by a concave portion (20) the profil of which corresponds to the outer profile of said ring member.

2. Holding device according to claim 1, characterized in that each rider (13) mounted on a ring member (6, 7) comprises in one of its flanges cooperating with one of the flat portions of the ring member a transverse bore (21) parallel to the flat portion and provided with an inner screwthread adapted to recive adustably a screw (22) the end of which protrudes into a hole perpendicular to said bore, said hole containing a stopping ball (25) protruding from said hole so as to project through a narrow orifice delimited by restriction of said hole, in such a manner that a displacement of said screw causes said ball to be blocked against said ring member while immobilizing said rider (13).

3. Holding device according to claim 1 or 2, characterized in that at least one flange (13a, 13b) of each rider (13) has an outer stud or a groove (36, 37) adapted to cooperate with a groove or stud, respectively, which presents the same profile and which is provided on a support (39) comprising a passage (40) containing a tubular element (41) provided with an outer screw thread and having a narrow axial channel (45) adapted to receive a rod or needle (3, 4) which is fixedly maintained with respect to said tubular element, said support further comprising a transverse recess (49) enclosing a captive nut (50) cooperating with the screw thread (42) of said tubular element, while said passage of the latter has an inner sliding element (52) slideably engaged in a radial groove (51) formed in the outer surface of the tubular element, the arrangement being such that rotation of said nut results in displacing said tubular element in said support, and in applying a tensile force or a compression force onto the corresponding rod or needle with respect to said bone element.

4. Holding device according to claim 3, characterized in that said rod or needle (3, 4) is immobilized in said narrow channel (45) of said tubular element (41) by a screw (47) inserted in a radial passage (48) of a head (43) mounted into the end of said tubular element.

5. Holding device according to any one of claims 1 to 4, characterized in that each rider (13) is adapted to be associated to a wedge member (27) adapted to be placed into one of the flanges (13a, 13b) of said rider, and in that said wedge member (27) is connected to said rider (13) by an axis (28) held by the wedge member and engaging a bore (29) provided in one flange of the rider and extending perpendicularly to the surface of said flat portions (14, 15) of the ring member (6, 7).

6. Holding device according to claim 5, characterized in that said wedge member (27) is immobilized with respect to the rider (13) by a radial locking screw (31) extending through a threaded bore (30) provided in the rider body and opening into the bore (29) receiving the axis (28) supported by said wedge member.

7. Holding device according to claim 6, characterized in that said axis (28) has an intermediate groove engaged by the end (32) of said locking screw (31).

8. Holding device according to any one of claims 1 to 7, wherein each ring member (6, 7) is constituted by two semicircular members (6a, 6b) adapted to be assembled by a set of two straight prolongators (53, 54) of adjustable length, characterized in that each prolongator (53, 54) has a cross-sectional profile identical to that of the ring member and comprises at its ends connecting members (55, 56) for connection to the mutually facing ends of each semicircular member (6a, 6b).

9. Holding device according to claim 8 characterized in that each connecting member is constituted by a protruding end portion (55, 56) provided on the semicircular member (6a, 6b) or the prolongator (53, 54) and introduced into a recess (57) provided in the prolongator or the semicircular member, respectively, said prolongator and said semicircular member being locked by at least one transverse screw (58) when said end portion has been introduced into said recess.

10. Holding device according to any one of claims 1 to 9, characterized in that each ring member (6, 7) is associated to an assembly (9) of twin collars constituted each by two pairs of jaws (59, 60) interconnected by a common transverse axis (61), one of said pairs of jaws being adapted to clamp the ring member and the other pair being adapted to clamp one end of an adjustable spacing piece (8) extending between the two parallel ring members (6, 7) perpendiculary to the respective planes thereof.

11. Holding device according to claim 10, characterized in that each spacing piece (8) is constituted by two elements (8a, 8b) extending each coaxially to the other element, said two elements being associated to an interposed connector assembly (10) comprising a rod the two opposed ends (10a, 10b) of which are threaded in mutually opposed directions and cooperate respectively with two nuts (67–68) integral with the ends of said spacing piece (8) that face each other.

12. Holding device according to claim 11, characterized in that one of two pairs of jaws of the twin collars is rendered integral with a bar (11) comprising successive passages (72) for fixing a rod or needle support mounted in front of the ring member (6, 7) in a cantilever fashion.

FIG. 1

FIG. 2

FIG. 8

FIG. 3

FIG. 4

FIG. 5

FIG. 6

FIG. 7